# EUROPÄISCHE PATENTSCHRIFT

(11) **EP 1 839 043 B1**
(45) Veröffentlichungstag und Bekanntmachung des Hinweises auf die Patenterteilung: **14.09.2011**
(21) Anmeldenummer: 06704688.8
(22) Anmeldetag: 10.01.2006
(51) Int. Cl.: G01N 25/72

(54) **VERFAHREN ZUR PRÜFUNG AUF NICKELSULFIDEINSCHLÜSSE IN EINSCHEIBENSICHERHEITSGLAS UND VORRICHTUNG HIERFÜR**
METHOD FOR EXAMINING THE PRESENCE OF NICKEL SULPHIDE INCLUSIONS IN TEMPERED SAFETY GLASS AND APPARATUS THEREFOR
PROCEDE DE VERIFICATION DE L'EVENTUELLE PRESENCE D'INCLUSIONS DE SULFURE DE NICKEL DANS DU VERRE TREMPE DE SURETE ET DISPOSITIF CORRESPONDANT

(30) Priorität: 12.01.2005 AT 392005
(43) Veröffentlichungstag der Anmeldung: 03.10.2007
(73) Patentinhaber: Euroglas GmbH, 39340 Haldensleben (DE)
(72) Erfinder: Schuller, Thomas, A-3335 Weyer (AT)
(74) Vertreter: Wirnsberger, Gernot
(86) Internationale Anmeldenummer: PCT/AT2006/000009
(87) Internationale Veröffentlichungsnummer: WO 2006/074494

(56) Entgegenhaltungen:
- BE-A1- 792 952
- US-A- 5 357 112
- US-A- 5 654 977
- PATENT ABSTRACTS OF JAPAN Bd. 2000, Nr. 21, 3. August 2001 (2001-08-03) -& JP 2001 089174 A (NIPPON SHEET GLASS CO LTD), 3. April 2001 (2001-04-03)

## Beschreibung

Die Erfindung betrifft ein Verfahren zur Prüfung auf Vorliegen von Nickelsulfideinschlüssen in einem Gegenstand aus Glas wie ein Einscheibensicherheitsglas.

Weiter hat die Erfindung eine Vorrichtung zur Prüfung eines Vorliegens von Nickelsulfideinschlüssen in einem Gegenstand aus Glas wie ein Einscheibensicherheitsglas zum Gegenstand.

Großflächige Glasscheiben wie sie beispielsweise in baulichen Konstruktionen eingesetzt werden, werden einer thermischen Behandlung unterworfen, um sie für Einsatzzwecke vorzuspannen bzw. zu härten. Mit einem Vorspannen bzw. einer Härtung von Glasscheiben soll eine Druckvorspannung aufgebracht werden, sodass an der Oberfläche einer Glasscheibe vorhandene Risse zusammengepresst werden, wodurch eine versagensfreie Gebrauchsdauer der Glasscheibe erhöht ist.

Für Einscheibensicherheitsgläser sind Vorspann- bzw. Härtungsprozesse von besonderer Wichtigkeit: Derartige Gläser werden beispielsweise im Bereich der Fassadenverglasungen eingesetzt und müssen höchste Forderungen nicht nur in Bezug auf eine gewünschte Bruchstruktur, sondern auch hinsichtlich einer langen Gebrauchsdauer bzw. eines Nichtauftretens von Materialversagen erfüllen.

Allerdings ist alleine durch ein Vorspannen bzw. Härten keineswegs eine lange Gebrauchsdauer bzw. eine hohe Materialstabilität garantiert, da bei der Herstellung in einem Glas nicht nur Risse auftreten können, sondern auch Fremdmaterialien eingeschlossen werden können, welche Ursache für Materialversagen sein können.

Konkret sind im Zusammenhang mit Glasscheiben und insbesondere Einscheibensicherheitsgläsern Nickelsulfideinschlüsse besonders unliebsame Einschlüsse. Ohne besondere Maßnahmen chemischer Natur können bei einer Herstellung von Glasscheiben durch Reduktion von über Ausgangsmaterialien eingebrachtem Natriumsulfat und aus Nickel, welches sich aus dem Edelstahl einer Produktionsvorrichtung löst, Nickelsulfidverbindungen (z. B. NiS- und/oder Ni₇S₆-Einschlüsse, wobei NiSₓ-Einschlüsse mit x = 1,0 bis 1,1 bruchkritisch sind) gebildet werden und folglich in fertig erstellten Glasgegenständen Nickelsulfideinschlüsse vorliegen. Wird ein Glasgegenstand mit Nickelsulfideinschlüssen nun vorgespannt bzw. gehärtet, das heißt auf erhöhte Temperaturen von mehr als 500 °C gebracht und anschließend verstärkt abgekühlt, beispielsweise durch Anblasen mit kalter Luft, so wandelt sich bei den erhöhten Temperaturen NiS in eine allotrope Hochtemperaturmodifikation (α-NiS) um, welche ab 379 °C stabil ist. Diese Hochtemperaturmodifikation wird beim verstärkten Abkühlen auf etwa Raumtemperatur "eingefroren" und wandelt sich bei Raumtemperatur im Zeitraum von Monaten und Jahren langsam in die bei Raum- bzw. Umgebungstemperatur thermodynamisch stabile β-NiS-Modifikation um, welche ein um circa 3 Volumenprozent größeres Volumen als die α-NiS-Modifikation hat. Durch die Volumenzunahme können im Inneren von beispielsweise Einscheibensicherheitsgläsern bei entsprechender Größe und Position der Einschlüsse derart große Spannungen auftreten, dass es nach Jahren zu einem spontanen Bruch bzw. unvorhersehbaren Materialversagen kommen kann.

Es versteht sich, dass Spontanbrüche von Gläsern, welche im baulichen Bereich oder in Kraftfahrzeugen eingesetzt sind, ein großes Gefahrenpotenzial darstellen und zu bedeutenden Personen- und Sachschäden führen können.

Aus dem Stand der Technik sind Bemühungen bekannt geworden, dieses Gefahrenpotenzial bei der Verwendung durch eine vorherige Prüfung zu verringern. So ist es aus der EP 0 853 069 B1 bekannt, Einscheibensicherheitsgläser nach einem Vorspannen bzw. Härten für mehrere Stunden einer weiteren Wärmebehandlung bei einer Temperatur von bis zu 300 °C zu unterwerfen. Bei diesen Temperaturen ist eine Umwandlung von α-NiS in β-NiS schneller als bei Raum- bzw. Umgebungstemperaturen, weshalb Scheiben mit großen Nickelsulfideinschlüssen und entsprechend hoher Wahrscheinlichkeit eines Spontanbruches bei später Verwendung diese Wärmebehandlung bzw. diesen Test mit einer bestimmten Wahrscheinlichkeit nicht überstehen und damit ausselektiert werden können.

Aus der JP 2001089174 A ist ein Verfahren zur Prüfung auf Nickelsulfideinschlüsse in Glas bekannt geworden, wobei nach einer Herstellung eines Glases eine erste Wärmebehandlung erfolgt, um α-NiS in β-NiS umzuwandeln, und wobei während dieser ersten Wärmebehandlung das Glas mit Röntgenstrahlen durchstrahlt wird, um Nickelsulfideinschlüsse bzw. deren Größe festzustellen. Ein Härten des Glases durch eine zweite Wärmebehandlung erfolgt nur dann, wenn eine Größe der Nickelsufideinschlüsse einen bestimmten Wert nicht übersteigt.

Der vorstehend dargestellte, sogenannte "heat-soak-test" gemäß der EP 0 853 069 B1 ist energieintensiv und dauert lange. Von diesem Stand der Technik ausgehend ist Ziel der Erfindung, ein Verfahren der eingangs genannten Art anzugeben, welches weniger energieintensiv und rascher durchführbar ist. Ein weiteres Ziel der Erfindung besteht in der Angabe einer Vorrichtung zur Prüfung eines Vorliegens von Nickelsulfideinschlüssen in einem Gegenstand aus Glas, mit welcher energieeffizient und rasch Einschlüsse aus Nickelsulfid nachgewiesen werden können.

Das verfahrensmäßige Ziel der Erfindung wird durch ein Verfahren gemäß Anspruch 1 erreicht. Vorteilhafte Varianten eines erfindungsgemäßen Verfahrens sind Gegenstand der Ansprüche 2 bis 6.

Ein besonderer Vorteil eines erfindungsgemäßen Verfahrens ist darin zu sehen, dass eine Prüfung auf Vorliegen von Nickelsulfideinschlüssen im Rahmen eines Vorspannens bzw. Härtens erfolgt. Am Ende des Vorspannens/Härtens ist eine Kenntnis über allfällige Nickelsulfideinschlüsse bereits gegeben und weitere Wärmebehandlungen als Qualitätstests sind nicht notwendig. Ein erfindungsgemäßes Verfahren kann daher rasch und energiesparend durchgeführt werden.

Bei der Erfindung wird die Erkenntnis ausgenützt, dass Nickelsulfideinschlüsse im Glas bei Temperaturen von mehr als 500 °C eine charakteristische Strahlung intensiv emittieren, wohingegen ein Gegenstand aus Glas wie ein Einscheibensicherheitsglas keine oder nur sehr intensitätsschwach Strahlung emittiert. Somit wird in Bereichen beispielsweise eines Einscheibensicherheitsglases, in welchem keine Nickelsulfideinschlüsse vorliegen, nur eine sehr schwache Strahlung beobachtet, während in Bereichen mit Nickelsulfideinschlüssen starke Emissionen registriert werden können.

Die Erfindung hat insbesondere auch den Vorteil, dass Nickelsulfideinschlüsse durch deren charakteristisches Emissionsspektrum direkt bzw. unmittelbar nachgewiesen werden können. Dies ermöglicht es beispielsweise, Größe der Einschlüsse, Lage der Einschlüsse im Glas und relative Häufigkeit der Einschlüsse sowie Abstand bzw. Anordnung einzelner Einschlüsse festzustellen. Da bereits einige wenige emittierte Photonen zur Registrierung ausreichend sind, können mit einem erfindungsgemäßen Verfahren Nickelsulfideinschlüsse im sub-Mikrometer-Bereich nachgewiesen werden. Dies erlaubt es, quantitative Kriterien für eine Verwendung bzw. Nichtverwendung vorgespannter Glasgegenstände festzusetzen.

In einer besonders günstigen Variante der Erfindung wird der Gegenstand auf eine Temperatur von zumindest 600 °C gebracht. Ausgehend von diesen Temperaturen kann einerseits eine gute Vorspannung bzw. eine gute Härtung erreicht werden und Risse im Glasgegenstand möglichst vollständig vermieden werden. Andererseits nimmt mit zunehmender Temperatur die Intensität einer Emission von Nickelsulfideinschlüssen zu und bei einer Temperatur über 600 °C können Nickelsulfideinschlüsse mit noch höherer Sicherheit aufgefunden werden. Um diese Wirkungen zu erreichen und dabei möglichst energiesparend vorzugehen, ist es günstig, wenn der Gegenstand auf eine Temperatur von 630 bis 680 °C gebracht wird.

Eine von Nickelsulfideinschlüssen emittierte Strahlung wird in günstiger Weise im Wellenlängenbereich von 800 bis 1200 nm registriert und ausgewertet. In diesem Wellenlängebereich sind Emissionen von einer Glasmatrix gering, weshalb bei der Registrierung von Strahlung ein Verhältnis von Emissionen von Nickelsulfideinschlüssen zu Emissionen der Glasmatrix hoch ist und bei einer Auswertung Nickelsulfideinschlüsse relativ zur gläsernen Matrix besonders deutlich hervortreten.

Emittierte Strahlung wird bevorzugt mit einer Charge-coupled-device-(CCD-)Kamera oder einer Complementary-metal-oxide-semiconductor-(CMOS-)Kamera registriert. Sowohl eine CCD (-Kamera als auch eine CMOS-Kamera ermöglichen es grundsätzlich, emittierte Strahlung innerhalb von Bruchteilen einer Sekunde aufzunehmen, sodass relativ zum Vorspann- bzw. Härtungsprozess, welcher zumindest mehrere Minuten dauert, Emissionen äußerst rasch erfasst und ausgewertet werden können.

Eine Auswertung von registrierter Strahlung wird bevorzugt mittels eines Computers durchgeführt. Insbesondere wenn eine der vorgenannten Kameras zum Einsatz kommt, kann ein Prüfen auf Vorliegen von Nickelsulfideinschlüssen rasch und vollständig automatisiert erfolgen.

In einer Variante der Erfindung ist es vorgesehen, dass der Gegenstand unter Bewegung auf eine Temperatur von zumindest 500 °C gebracht wird, beispielsweise in einem Durchlaufofen, und nach Erreichen der Temperatur unter fortdauernder Bewegung des Gegenstandes bereichsweise ein Registrieren emittierter Strahlung erfolgt. In dieser Weise kann ein Glasgegenstand, insbesondere ein Einscheibensicherheitsglas, besonders rasch vorgespannt und auf Vorliegen von Nickelsulfideinschlüssen geprüft werden.

Das weitere Ziel der Erfindung eine Vorrichtung zur Prüfung eines Vorliegens von Nickelsulfideinschlüssen in einem Gegenstand aus Glas anzugeben, mit welcher energieeffizient und rasch Einschlüsse aus Nickelsulfid nachgewiesen werden können, wird durch eine Vorrichtung gemäß Anspruch 7 gelöst. Vorteilhafte Weiterbildungen einer erfindungsgemäßen Vorrichtung sind Gegenstand der Ansprüche 8 bis 12.

Mit einer erfindungsgemäßen Vorrichtung kann in einfacher und energiesparender Weise eine Prüfung auf Vorliegen von Nickelsulfideinschlüssen in Glasgegenständen erfolgen. Eine geeignet ausgelegte Dunkelkammer dient dazu, einen im Rahmen eines Vorspann- bzw. Härteprozesses auf eine Temperatur von zumindest 500 °C erwärmten Gegenstand aus Glas in einem dunklen Raum unter Ausschluss von gegenstandsfremder Strahlung aufzunehmen. Über eine mit der Dunkelkammer verbundene Einrichtung lassen sich Emissionen von Nickelsulfideinschlüssen, welche aus einer emissionsschwachen Glasmatrix quasi herausleuchten, registrieren.

Ein anderer Vorteil einer erfindungsgemäßen Vorrichtung ist die Möglichkeit, Nickelsulfideinschlüsse direkt bzw. unmittelbar zu beobachten, was eine Aussagesicherheit in Bezug auf mögliche zukünftige Spontanschäden eines vorgespannten Gegenstandes aus Glas, insbesondere eines Einscheibensicherheitsglases, erhöht.

Da eine von Nickelsulfideinschlüssen emittierte Strahlung an sich innerhalb kurzer Zeit registriert werden kann, kann mit einer erfindungsgemäßen Vorrichtung äußerst schnell auf Vorliegen von Nickelsulfideinschlüssen geprüft werden.

In einer vorteilhaften Weiterbildung der Erfindung ist die Dunkelkammer kühlbar. Die Dunkelkammer kann in diesem Fall auch bei Aufnahme von mehrere hundert Grad Celsius heißen Gegenständen auf konstanter Temperatur gehalten werden, sodass konstante Bedingungen für eine Registrierung emittierter Strahlung sichergestellt und thermisch bedingte Störsignale vermieden sind.

Wenn die Einrichtung eine CCD-Kamera oder eine CMOS-Kamera umfasst, kann eine rasche Registrierung emittierter Strahlung innerhalb von Sekundenbruchteilen erfolgen und ein zu prüfender Gegenstand aus Glas braucht lediglich für diese Zeitspanne auf einer Härtetemperatur gehalten werden.

Eine rasche Messung ist vor allem auch dann von Vorteil, wenn eine bereichsweise Prüfung erforderlich sein kann, beispielsweise bei großflächigen Glasscheiben. Insbesondere für derartige Anwendungen ist es günstig, wenn bei einer erfindungsgemäßen Vorrichtung die CCD-Kamera bzw. die CMOS-Kamera mit einem Computer in Verbindung steht, um eine rasche und automatisierte Prüfung zu ermöglichen.

Die Dunkelkammer kann innen mit einem schwarzen Hochtemperaturlack beschichtet sein, was im Hinblick auf ein Registrieren sehr schwacher Emissionen, beispielsweise bei Vorliegen sehr kleiner Nickelsulfideinschlüsse, Vorteile bringt. Um eine raue Beschichtung zu erreichen und damit ungewollte Reflexionen in der Dunkelkammer zu vermeiden, ist es günstig, wenn diese Beschichtung durch Aufsprühen oder Pulverbeschichten erstellt ist.

Weitere Vorteile der Erfindung ergeben sich aus dem Zusammenhang der Beschreibung und den Ausführungsbeispielen.

Im Folgenden ist die Erfindung anhand eines Ausführungsbeispieles noch weitergehend dargestellt.

Es zeigen:
Figur 1 einen Abschnitt einer Vorrichtung zum Vorspannen eines Einscheibensicherheitsglases mit integrierter Prüfvorrichtung zum Nachweis von Nickelsulfideinschlüssen;
Figur 2 eine fotografische Aufnahme eines Teilbereiches eines Einscheibensicherheitsglases in einer Dunkelkammer (schematisch);
Figur 3 Ausschnitte aus Emissionsspektren, welche an Positionen A bzw. B gemäß Figur 2 ermittelt wurden.

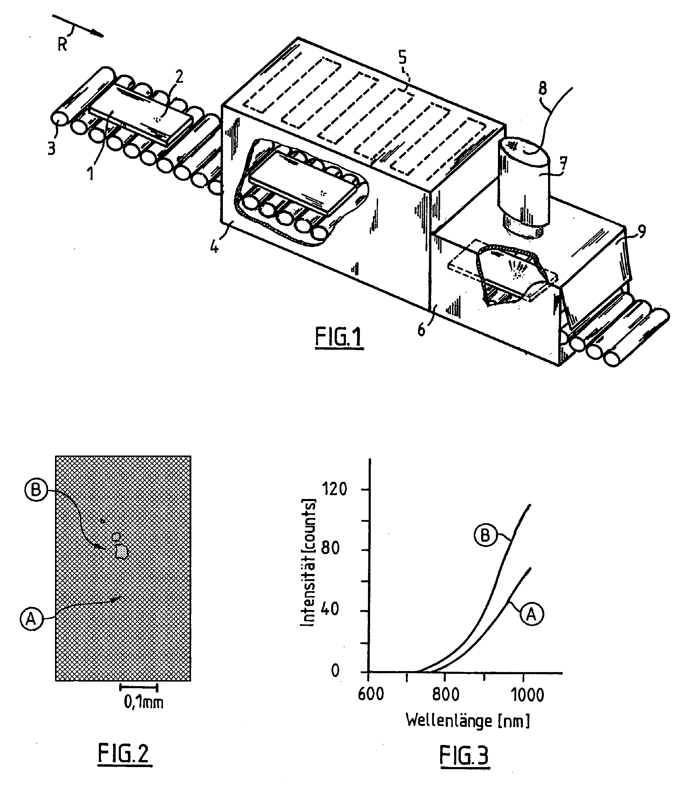

In Figur **1** ist ein Abschnitt einer erfindungsgemäßen Vorrichtung dargestellt, welche bei einem kontinuierlichen Vorspannen bzw. Härten von Einscheibensicherheitsgläsern Anwendung findet. Die Vorrichtung weist ein Fördermittel **3** auf, mit der ein Einscheibensicherheitsglas **1**, welches produktionstechnisch bedingt Nickelsulfideinschlüsse **2** aufweist, in Richtung **R** zu einem Durchlaufofen **4** transportiert und durch diesen hindurchgeführt wird. Das Fördermittel 3 kann beliebig ausgebildet sein, beispielsweise als Förderband oder als aufeinanderfolgende, drehbare Rollen, sofern der Transport zum und durch den Durchlaufofen **4** ermöglicht ist. Der Durchlaufofen **4** ist mit zumindest einem Heizelement **5** ausgestattet, durch welches im Durchlaufofen **4** eine gewünschte Temperatur, beispielsweise 650 °C, konstant eingestellt werden kann. Ein zu härtendes Einscheibensicherheitsglas **1** wird bei Transport durch den Durchlaufofen **4** auf eine Härtetemperatur erwärmt, sodass das Einscheibensicherheitsglas **1** spätestens beim Verlassen des Durchlaufofens **4** auf eine für ein Vorspannen/Härten erforderliche Temperatur gebracht ist.

An den Durchlaufofen **4** schließt eine Dunkelkammer **6** an, in welche ein Einscheibensicherheitsglas **1** nach Verlassen des Durchlaufofens **4** gefördert wird, ohne dass eine Abkühlung unter 500 °C stattfindet. Die Dunkelkammer **6** ist mit nicht eigens dargestellten Mitteln einerseits gegen Eindringen von Licht aus dem Durchlaufofen **4** und andererseits gegen Eindringen von Licht aus ihrem Auslassbereich **9** geschützt. Dadurch kann ein Einscheibensicherheitsglas 1 mit einer Härtetemperatur von 500 °C oder mehr in einem völlig dunklen Raum in Bezug auf Emissionen beobachtet werden.

Eine Beobachtung emittierter Strahlung kann mit einer beliebigen Einrichtung durchgeführt werden, mit welcher Photonen bzw. elektromagnetische Strahlung registriert werden kann. Beispielsweise kann eine CCD-Kamera **7**, eine Fotokamera, oder ein Spektrometer verwendet werden. Um eine Anbindung der Beobachtungseinrichtung an die Dunkelkammer **6** zu ermöglichen, weist diese eine Öffnung auf, über welche vom Einscheibensicherheitsglas **1** emittierte Strahlung austreten kann.

Eine Anbindung kann, wie in Figur **1** dargestellt, erfolgen, indem eine CCD-Kamera **7** unmittelbar an einer Dunkelkammer **6** befestigt ist und direkt Licht aus dem Inneren der Dunkelkammer **6** zugeleitet bekommt. Alternativ kann eine Anbindung auch durch einen oder mehrere Lichtwellenleiter erfolgen. In diesem Fall kann eine Beobachtungseinrichtung, beispielsweise eine CCD-Kamera **7** in Entfernung zur Dunkelkammer **6** aufgestellt werden, und emittierte Strahlung bzw. Licht, welches aus der Öffnung einer Dunkelkammer **6** austritt, wird über Lichtwellenleiter zum Eintrittsspalt einer CCD-Kamera **7** geleitet.

Sollen sowohl Bilder für eine bildverarbeitende Auswertung aufgenommen werden, als auch spektrochemische Analysen durchgeführt werden, so kann eine erfindungsgemäße Vorrichtung neben einer CCD- oder CMOS-Kamera auch ein Spektrofotometer umfassen.

Die mit Hilfe einer CCD-Kamera **7** registrierten Daten werden über eine Datenleitung **8** einer Auswerteeinheit, z. B. einem Computer, zugeführt und vorzugsweise automatisiert ausgewertet.

Ein Beispiel einer derartigen Auswertung ist in den Figuren 2 und 3 näher dargestellt. Figur 2 zeigt eine fotografische Aufnahme (schematisch) eines Bereiches eines Einscheibensicherheitsglases **1**, wie sie bei einer Beobachtung in einer Dunkelkammer bei ca. 620 °C erhalten wird. Überwiegend wird ein dunkles bzw. schwarzes Bild erhalten, z. B. an der Stelle **A**, da Glas bei diesen Temperaturen nur sehr wenig sichtbare Strahlung emittiert. An der Stelle **B**, welche zu einem Nickelsulfideinschluss im Einscheibensicherheitsglas **1** korrespondiert, wird hingegen ein rotes Bild erhalten (in Figur 2 als gepunkteter dargestellt).

Zu den Stellen **A** bzw. **B** in Figur 2 entsprechen in Figur 3 die mit einem optischen Spektrometer erhaltenen Spektralkurven. Diese Spektralkurven zeigen, dass am langwelligen Ende des sichtbaren elektromagnetischen Spektrums die Emission eines Nickelsulfidpartikels wesentlich größer ist als ein Blindwert des Glases. Das bedeutet, dass selbst bei sehr kleinen Partikeln, wie sie in Figur 2 gezeigt sind, ein Nachweis und Bestimmung von Größe und Lage von Nickelsulfideinschlüssen ohne Weiteres möglich ist.

Zur Durchführung einer spektrofotometrische Analyse genügt es grundsätzlich, Emissionen eines Einscheibensicherheitsglases an einer bestimmten Wellenlängenposition zu beobachten, beispielsweise bei 800 nm. Günstig ist es auch, im nahen Infrarot, z. B. bei 1200 nm emittierte Strahlung zu registrieren, da für diesen Bereich einerseits kostengünstige Detektoren auf Siliciumbasis bekannt sind und andererseits ein Quotient von Emission von Nickelsulfid/Emission von Glas ein Maximum beträgt. Anders ausgedrückt: Nickelsulfideinschlüsse heben sich bei einer Auswertung besonders deutlich von einer Glasmatrix ab.

Bringt eine gegebenenfalls automatisierte Auswertung von emittierter Strahlung als Ergebnis, dass zu viele und/oder zu große Nickelsulfideinschlüsse bezogen auf eine Einschlusstiefe vorliegen, so wird das Einscheibensicherheitsglas **1** ausgeschieden bzw. nicht eingesetzt. Zweckmäßig ist es dann, ein Einscheibensicherheitsglas **1** unmittelbar nach Verlassen einer Dunkelkammer **6** vom Fördermittel **3** zu entfernen und lediglich brauchbare Einscheibensicherheitsgläser **1** durch eine der Dunkelkammer **6** nachgeschaltete Kühleinrichtung verstärkt abzukühlen. Zu diesem Zweck kann das Fördermittel **3** auch eine Verzweigungsstelle aufweisen, an der Einscheibensicherheitsgläser **1** je nach Ergebnis einer Prüfung entweder zu einer Abkühlvorrichtung oder zu einer Ausschusssammeleinrichtung befördert werden. Eine derartige Maßnahme leistet einen weiteren Beitrag in Hinblick auf eine hohe Energieeffizienz, da ausschließlich brauchbare Einscheibensicherheitsgläser **1** gekühlt werden müssen.

Wie in Figur 1 gezeigt ist, kann eine Dunkelkammer **6** unmittelbar mit einem Durchlaufofen **4** verbunden sein und ein Fördermittel **3** zum Transport eines Einscheibensicherheitsglases **1** durch beide Einrichtungen vorgesehen sein. Dies hat den Vorteil, dass ein kontinuierliches Vorspannen einschließlich Prüfung auf Vorliegen von Nickelsulfideinschlüssen erfolgen kann. Alternativ dazu können Härteofen und Dunkelkammer auch voneinander getrennt und beispielsweise nebeneinander aufgestellt sein. In diesem Fall können mehrere Einscheibensicherheitsgläser gleichzeitig auf Härtetemperatur erhitzt werden, was in Hinblick auf eine bedarfsorientierte und energieeffiziente Herstellung vorteilhaft sein kann.

## Patentansprüche

1. Verfahren zur Prüfung auf Vorliegen von Nickelsulfideinschlüssen (2) in einem Gegenstand aus Glas wie ein Einscheibensicherheitsglas (1) während eines Vorspannens bzw. Härtens desselben, wobei der Gegenstand auf eine Temperatur von zumindest 500 °C gebracht wird und bei dieser Temperatur eine vom Gegenstand emittierte elektromagnetische Strahlung registriert und ausgewertet wird, wonach der Gegenstand verstärkt abgekühlt wird, um ihn vorzuspannen bzw. zu härten.

2. Verfahren nach Anspruch 1, wobei der Gegenstand auf eine Temperatur von zumindest 600 °C, insbesondere 630 bis 680 °C, gebracht wird.

3. Verfahren nach Anspruch 1 oder 2, wobei emittierte Strahlung im Wellenlängenbereich von 800 bis 1200 nm registriert und ausgewertet wird.

4. Verfahren nach einem der Ansprüche 1 bis 3, wobei emittierte Strahlung mit einer CCD-Kamera (7) oder einer CMOS-Kamera registriert wird.

5. Verfahren nach einem der Ansprüche 1 bis 4, wobei eine Auswertung mittels eines Computers durchgeführt wird.

6. Verfahren nach einem der Ansprüche 1 bis 5, wobei der Gegenstand unter Bewegung auf eine Temperatur von zumindest 500 °C gebracht wird, beispielsweise in einem Durchlaufofen (4), und nach Erreichen der Temperatur unter fortdauernder Bewegung des Gegenstandes bereichsweise ein Registrieren emittierter Strahlung erfolgt.

7. Glasprüfvorrichtung zum Prüfen eines Vorliegens von Nickelsulfideinschlüssen (2) in einem Gegenstand aus Glas wie ein Einscheibensicherheitsglas (1), umfassend eine Dunkelkammer (6) zur Aufnahme eines eine Temperatur von zumindest 500 °C aufweisenden Gegenstandes aus Glas und eine mit der Dunkelkammer (6) verbundene Einrichtung, mit welcher eine vom Gegenstand bei der genannten Temperatur emittierte Strahlung registrierbar ist.

8. Glasprüfvorrichtung nach Anspruch 7, wobei die Dunkelkammer (6) kühlbar ist.

9. Glasprüfvorrichtung nach einem der Ansprüche 7 oder 8, wobei die Einrichtung eine CCD-Kamera (7) oder eine CMOS-Kamera umfasst.

10. Glasprüfvorrichtung nach Anspruch 9, wobei die CCD-Kamera (7) bzw. die CMOS-Kamera mit einem Computer in Verbindung steht.

11. Glasprüfvorrichtung nach einem der Ansprüche 7 bis 10, wobei die Dunkelkammer (6) innen mit einem schwarzen Hochtemperaturlack beschichtet ist.

12. Glasprüfvorrichtung nach Anspruch 11, wobei die Beschichtung durch Aufsprühen oder Pulverbeschichten erstellt ist.

## Claims

1. A method for testing for the presence of nickel sulphide inclusions (2) in an object made of glass such as a toughened safety glass (1) during toughening or hardening thereof, wherein the object is brought to a temperature of at least 500°C and electromagnetic radiation emitted by the object at this temperature is recorded and evaluated, after which the object is abruptly cooled in order to toughened or harden it.

2. The method according to claim 1, wherein the object is brought to a temperature of at least 600°C, in particular 630 to 680°C.

3. The method according to claim 1 or 2, wherein ascertained radiation in the wavelength range from 800 to 1200 nm is recorded and evaluated.

4. The method according to any one of claims 1 to 3, wherein emitted radiation is recorded with a CCD camera (7) or a CMOS camera.

5. The method according to any one of claims 1 to 4, wherein an evaluation is carried out by means of a computer.

6. The method according to any one of claims 1 to 5, wherein the object is brought to a temperature of at least 500°C whilst moving, for example in a through-type furnace (4), and recording of the ascertained radiation takes place in zones after the temperature is reached with continuous movement of the object.

7. A glass testing device for testing for the presence of nickel sulphide inclusions (2) in an object made of glass such as a toughened safety glass (1), comprising a darkroom (6) for photographing an object made of glass having a temperature of at least 500°C and a device which is connected to the darkroom (6) and with which radiation emitted by the object at the said temperature can be recorded.

8. The glass testing device according to claim 7, wherein that the darkroom (6) can be cooled.

9. The glass testing device according to any one of claims 7 or 8, wherein the device comprises a CCD camera (7) or a CMOS camera.

10. The glass testing device according to claim 9, wherein the CCD camera (7) or the CMOS camera is connected to a computer.

11. The glass testing device according to any one of claims 7 to 10, wherein the darkroom (6) is coated internally with a black high-temperature lacquer.

12. The glass testing device according to claim 11, wherein the coating is prepared by spraying or powder coating.

## Revendications

1. Procédé de vérification de la présence d'inclusions de sulfure de nickel (2) dans un objet en verre, tel que par exemple un verre de sécurité trempé (1) pendant une précontrainte ou une trempe de ce dernier, l'objet étant amené à une température d'au moins 500 °C et à cette température, un rayonnement électromagnétique émis par l'objet étant enregistré et évalué, suite à quoi, l'objet étant refroidi de façon renforcée, pour le précontraindre ou le tremper.

2. Procédé selon la revendication 1, l'objet étant amené à une température d'au moins 600°C, notamment de 630 à 680°C.

3. Procédé selon la revendication 1 ou 2, un rayonnement émis dans la gamme de longueur d'ondes de 800 à 1200 nm étant enregistré et évalué.

4. Procédé selon l'une quelconque des revendications 1 à 3, un rayonnement émis étant enregistré à l'aide d'une caméra CCD (7) ou d'une caméra CMOS.

5. Procédé selon l'une quelconque des revendications 1 à 4, une évaluation étant réalisée à l'aide d'un ordinateur.

6. Procédé selon l'une quelconque des revendications 1 à 5, l'objet étant amené sous déplacement à une température d'au moins 500 °C, par exemple dans un four à passage continu (4) et après atteinte de la température sous un déplacement continu de l'objet, un enregistrement du rayonnement émis étant effectué par zones.

7. Dispositif de contrôle de verre pour vérifier la présence d'inclusions de sulfure de nickel (2) dans un objet en verre, tel qu'un verre de sécurité trempé (1), comprenant une chambre noire (6) pour la réception d'un objet en verre présentant une température d'au moins 500 °C et avec un système relié à la chambre noire (6), à l'aide duquel un rayonnement émis par l'objet à la température citée est enregistrable.

8. Dispositif de contrôle de verre selon la revendication 7, la chambre noire (6) pouvant être refroidie.

9. Dispositif de contrôle de verre selon l'une quelconque des revendications 7 ou 8, le système comprenant une caméra CCD (7) ou une caméra CMOS.

10. Dispositif de contrôle de verre selon la revendication 9, la caméra CCD (7) ou la caméra CMOS étant connectée à un ordinateur.

11. Dispositif de contrôle de verre selon l'une quelconque des revendications 7 à 10, la chambre noire (6) étant revêtue à l'intérieur d'une laque haute température.

12. Dispositif de contrôle de verre selon la revendication 11, le revêtement étant effectué par vaporisation ou par poudrage.
